# EUROPEAN PATENT SPECIFICATION

(11) **EP 1 477 189 B1**
(45) Date of publication and mention of the grant of the patent: **19.12.2018**
(21) Application number: 03701858.7
(22) Date of filing: 24.01.2003
(51) Int. Cl.: A61L 15/58, A61K 9/70, C09J 7/00, C08J 3/24, C08J 7/00

(54) **PROCESS FOR PRODUCTION OF PRESSURE-SENSITIVE ADHESIVE MOLDINGS COMPRISING CROSSLINKED POLYMERS AS THE MAIN COMPONENT**
VERFAHREN ZUR HERSTELLUNG VON DRUCKEMPFINDLICHEN KLEBEFORMEN MIT VERNETZTEN POLYMEREN ALS HAUPTKOMPONENTE
PROCESSUS DE PRODUCTION DE MOULAGES D'ADHESIF AUTOCOLLANT CONSTITUE DE POLYMERES RETICULES COMME COMPOSANT PRINCIPAL

(30) Priority: 25.01.2002 JP 2002016796
(43) Date of publication of application: 17.11.2004
(73) Proprietor: Hisamitsu Pharmaceutical Co., Inc., Tosu-shi, Saga 841-0017 (JP)
(72) Inventor: YASUKOCHI, Takashi, Tsukuba-shi, Ibaraki 305-0856 (JP); YAMAGUCHI, Toshiro, Tsukuba-shi, Ibaraki 305-0856 (JP); TATEISHI, Tetsuro, Tsukkuba-shi, Ibaraki 305-0856 (JP); HIGO, Naruhito, Tsukuba-shi, Ibaraki 305-0856 (JP)
(74) Representative: Dr. Gassner & Partner mbB
(86) International application number: PCT/JP2003/000648
(87) International publication number: WO 2003/061719

(56) References cited:
- EP-A1- 0 402 291
- EP-A1- 0 629 639
- EP-A2- 0 793 972
- JP-A- 62 111 918
- JP-A- 63 150 222
- JP-A- 2002 241 732
- US-A- 3 532 708
- US-A- 3 769 254

## Description

### Technical Field

The present invention relates to a process for the production of a pressure-sensitive adhesive shaped product and a matrix preparation comprising a drug and a crosslinked polymer as a main component.

### Background Art

A pressure-sensitive adhesive layer constituting a patch normally comprises a mixture containing a drug and, as a main component, a polymer, and with regard to means for improving the tackiness and cohesiveness of this mixture, various techniques have been carried out in which a crosslinked polymer is formed by adding an appropriate crosslinking agent during the formulation process so as to gel the pressure-sensitive adhesive layer. Such a pressure-sensitive adhesive can be obtained by a method in which a drug, etc. is added to a crosslinked polymer, but in order to add a sufficient amount of drug to the pressure-sensitive adhesive layer and solve problems in shaping, a method in which an appropriate crosslinking agent is added during the formulation process to a mixture containing a drug and, as a main component, a polymer so as to gel the mixture is widely employed.

In particular, since many of the drugs used in patches are lipophilic, various types of lipophilic acrylic polymers containing substantially no water are used as the polymer constituting the pressure-sensitive adhesive layer. In recent years in particular, this type of nonaqueous patch often contains some liquid substance as a component to improve the skin permeability of the drug, but this further degrades the tackiness and cohesiveness of the pressure-sensitive adhesive layer, and crosslinking of the polymer is therefore a very important object.

Crosslinking of the polymer is generally carried out by a reaction between an appropriate crosslinking agent and a crosslinking functional group of the polymer. When crosslinking is carried out after a drug is mixed as described above, it is necessary for the reaction to proceed in a short period of time under mild conditions so as not to affect the stability of the drug. In such a case, even during shaping of a pressure-sensitive adhesive shaped product at room temperature, the crosslinking reaction often proceeds at a rate that is sufficient to hinder the shaping, and as a result the crosslinking might form a nonuniform polymer, the flowability might be lost during shaping, and shaping itself might become impossible, which is a problem.

US 3,532,708 A, US 3,769,254, EP 0 402 291 A1 and EP 0 629 639 A1 describe methods for the preparation of a pressure-sensitive adhesive from an interpolymer solution.

EP 0 793 972 A2 describes a process for the production of a pressure-sensitive adhesive shaped product wherein the pressure-sensitive adhesive contains a drug for percutaneous absorption use. The pressure-sensitive adhesive contains an acrylic copolymer and crosslinked acrylic copolymer particles.

An object of the present invention is thus to solve the above-mentioned problems of the art, and to provide a process for the production of a pressure-sensitive adhesive shaped product comprising a drug, the process enabling shaping of a crosslinked polymer to be carried out properly.

### Disclosure of Invention

As a result of an intensive investigation by the present inventors in order to solve the above-mentioned problems, it has been found that use of a crosslinking agent dissolved in a lower alcohol such as methanol or ethanol during the production of a pressure-sensitive adhesive shaped product enables the crosslinking rate to be controlled appropriately, and the crosslinking reaction can be carried out by drying and removing the alcohol after shaping into a desired shape, and as a result of a further investigation, the present invention has been accomplished.

The above object is solved by the features of claims 1 and 7.

The present invention relates to a process for the production of a pressure-sensitive adhesive shaped product comprising a drug, the process comprising mixing, in the presence of a lower alcohol, a hydroxyl group- or carboxyl group containing polymer and one or more crosslinking agents selected from the group consisting of metal alcoholates and boron-containing compounds, and then shaping either simultaneously with or followed by thermal crosslinking.

Furthermore, the present invention relates to a process for the production of a matrix preparation comprising a drug, the process comprising preparing a pressure-sensitive adhesive shaped product according to one of the Claims 1 to 6, wherein shaping is done by spreading the mixture on a film and the shaping is followed by thermal crosslinking, wherein the crosslinking is either simultaneously with or followed by laminating to a support.

Moreover, the present invention relates to the production process wherein the crosslinking agent is dissolved in the lower alcohol in advance.

Furthermore, the present invention relates to the production process wherein the polymer is dissolved in the lower alcohol in advance.

Moreover, the present invention relates to the production process wherein the crosslinking functional group is a hydroxyl group, and the crosslinking agent is boric acid.

Furthermore, the present invention relates to the production process wherein the polymer is an acrylic polymer or a methacrylic polymer.

Moreover, the present invention relates to a matrix preparation produced by the process above.

Furthermore, the present invention relates to the matrix preparation wherein the pressure-sensitive adhesive shaped product contains substantially no water.

In accordance with the present invention, since the lower alcohol inhibits the crosslinking reaction, the crosslinking reaction is substantially suppressed when shaping into a desired shape such as a sheet, and the crosslinking reaction subsequently proceeds rapidly as the lower alcohol is removed. Since a low boiling point lower alcohol is used, crosslinking can be carried out under mild heating conditions so that a drug does not decompose and, furthermore, since in the present invention it is unnecessary to use water as a solvent for a polymer mixture or a crosslinking agent solution, the present invention is suitable for a pressure-sensitive adhesive shaped product containing substantially no water, which is desirable as a plaster of a patch.

In the present description, 'containing substantially no water' means that no water is used in the production of the pressure-sensitive adhesive shaped product, or that the pressure-sensitive adhesive shaped product that is produced contains no water.

### Modes for Carrying Out the Invention

A crosslinkable monomer unit of a polymer used in the present invention is not particularly limited as long as it is an lipophilic monomer such as an acrylic or methacrylic monomer and has at least one hydroxyl or carboxyl group in the unit as a crosslinking functional group.

Specific examples thereof include hydroxyl group-containing acrylate monomers such as 2-hydroxyethyl acrylate, 3-hydroxypropyl acrylate, and 4-hydroxybutyl acrylate, hydroxyl group-containing methacrylate monomers such as 2-hydroxyethyl methacrylate, 3-hydroxypropyl methacrylate, and 4-hydroxybutyl methacrylate, vinyl alcohol, allyl alcohol, 3-buten-1-ol, 3-buten-2-ol, acrylic acid, methacrylic acid, itaconic acid, and maleic acid.

In particular, from the viewpoint of low reactivity, generally low skin irritation such as reddening or edema caused by residual functional groups, and suitability as a patch, which needs to be affixed to the skin for a long period of time, a hydroxyl group-containing acrylic monomer and a hydroxyl group-containing methacrylic monomer are preferable, and 2-hydroxyethyl acrylate is particularly preferable.

The hydroxyl group-containing monomer and/or carboxyl group-containing monomer can be used singly or in a combination of two or more kinds.

It is preferable for the acrylic monomer or the methacrylic monomer to be the main component of the polymer in the present invention, and the acrylic monomer or methacrylic monomer is contained at at least 30 wt % relative to the polymer, preferably 30 to 90 wt %, and particularly preferably 50 to 90 wt %.

The polymer used in the present invention is not particularly limited as long as it contains a hydroxyl or carboxyl group, and it may be a polymer of a single monomer or it may be a copolymer, but a copolymer is particularly preferable. Specific examples thereof include a copolymer of 2-hydroxyethyl acrylate, 2-ethylhexyl acrylate, and N-vinyl-2-pyrrolidone.

When the polymer is a copolymer, monomer units other than those at the crosslinking sites are not particularly limited, and specific examples of such monomers include acrylic acid, acrylonitrile, acrylic and methacrylic acid straight-chain alkyl esters such as methyl, ethyl, propyl, butyl, pentyl, hexyl, heptyl, octyl, nonyl, decyl, undecyl, dodecyl, and tridecyl esters, branched alkyl esters such as 2-ethylhexyl ester, substituted alkyl esters such as 2-hydroxyethyl, 3-hydroxypropyl, and 4-hydroxybutyl esters, vinyl acetate, vinylpyrrolidone, N-vinyl-2-pyrrolidone, allylamine, styrene, reactive polymers (macro monomers), vinyl propionate, methylvinylpyrrolidone, vinylpyridine, vinylpiperidone, vinylpiperazine, vinylpyrazine, vinylpyrrole, vinylimidazole, vinylcaprolactam, vinyloxazole, vinylmorpholine, 2-ethylhexyl acrylate, and methoxyethyl acrylate. 2-Ethylhexyl acrylate and vinylpyrrolidone are particularly preferable. These monomers can be used singly or in a combination of two or more kinds.

The crosslinking agent used in the present invention can be either a boron-containing compound or a metal alcoholate, but the boron-containing compound is preferable from the viewpoint of safety and drug stability. With regard to the boron-containing compound, boric acid and derivatives thereof in which the boron is +3 valent can be cited. Examples of the boric acid derivatives include a borate and a borate ester. With regard to the borate, there can be cited chemically acceptable inorganic and organic salts whose condensation number is not limited as long as the boron is +3 valent. Specific examples thereof include sodium tetraborate and ammonium borate. Examples of the borate ester include methyl borate, ethyl borate, propyl borate, and butyl borate. Boric acid is particularly preferable. These compounds can be anhydrous compounds or hydrates, and the anhydrous compounds are preferable.

These boron-containing compounds as the crosslinking agent are preferably added at 0.01 to 20 wt % relative to the total weight of the composition of the pressure-sensitive adhesive shaped product, and are more preferably added at 0.1 to 10 wt %, and particularly preferably 0.1 to 5 wt %, while taking into consideration the physical properties and skin irritation of the pressure-sensitive adhesive shaped product.

Examples of the metal alcoholate as the crosslinking agent of the present invention include titanium derivatives (titanium ethoxide, titanium butoxide, titanium methoxide, titanium propoxide, titanium isopropoxide, etc.); and aluminum derivatives (aluminum alcoholates (aluminum isopropoxide, aluminum ethoxide, aluminum butoxide, aluminum sec-butoxide, aluminum tert-butoxide, etc.)).

The metal alcoholate as the crosslinking agent is preferably added at 0.1 to 10 wt % relative to the total weight of the composition of the pressure-sensitive adhesive shaped product, more preferably 0.1 to 5 wt %, and particularly preferably 0.1 to 3 wt %, while taking into consideration the physical properties and skin irritation of the pressure-sensitive adhesive shaped product and the preparation.

The lower alcohol used in the present invention is an alcohol having 1 to 5 carbons and can be a monohydric, dihydric, or trihydric alcohol as long as it can dissolve the boron-containing compound. Specific examples thereof include methanol, ethanol, 1-propanol, 2-propanol, and 1-butanol, and methanol and ethanol are particularly preferable. They are used as solvents for the crosslinking agent solution or as solvents for the polymer solution, and can be used singly or in a combination of two or more kinds, or in combination with another solvent that is miscible with these alcohols, while taking into consideration the polymer crosslinking efficiency.

When a pressure-sensitive adhesive shaped product is produced in accordance with the present invention, mixing of the polymer and the crosslinking agent is carried out, for example, by dissolving the crosslinking agent in advance in a solvent containing a lower alcohol and adding the solution in which the crosslinking agent is dissolved to a mixture containing the polymer, or by adding the crosslinking agent to a solution containing a lower alcohol and the polymer. Subsequently, the mixture of the polymer and the crosslinking agent thus obtained is shaped into any desired shape such as a sheet shape, a tape shape, or a rod shape, and then thermally crosslinked. The thermal crosslinking can also be carried out simultaneously with the shaping, as in a case in which the mixture is poured into a heated mold.

The pressure-sensitive adhesive shaped product of the present invention needs no water during its production; even when water is used, the water can be removed by heating, and it is possible to obtain a pressure-sensitive adhesive shaped product containing substantially no water.

The pressure-sensitive adhesive shaped product comprising a drug can be shaped into any shape to suit the intended use, and typical examples of applications include medical patches and plasters

The production process of the present invention is explained below with reference to an example of the production of a matrix preparation for a patch.

A polymer is produced by polymerizing a hydroxyl group- or carboxyl group-containing nonaqueous monomer and/or various types of other monomers at an appropriate ratio in accordance with a standard method. It is also possible to use as a starting material a commercial product such as DURO-TAK ® (manufactured by National Starch and Chemical Company), PE-300 (manufactured by Nippon Carbide Industries Co., Inc.), or GELVA (trade name)(manufactured by Monsanto).

In order for the polymer and the crosslinking agent to be mixed in the presence of a lower alcohol, the polymer and the lower alcohol are mixed in advance, or the crosslinking agent and the lower alcohol are mixed in advance. A drug can be further mixed with the mixture thus obtained; it is preferable to add the drug prior to shaping, and more preferably prior to addition of the crosslinking agent.

The mixture thus obtained is shaped into, for example, a sheet shape by spreading it on a silicon-treated film.

The mixture shaped into the sheet shape by spreading is then heated so as to allow a crosslinking reaction to proceed. Heating is usually carried out at 60°C to 150°C for approximately 15 minutes to 1 hour. When the heating temperature is too high or the heating time is too long, there is a possibility that the drug might be decomposed or denatured. On the other hand, when the heating temperature is too low or the heating time is too short, the lower alcohol might remain, thus preventing the crosslinking reaction from proceeding sufficiently.

The pressure-sensitive adhesive shaped product obtained by thermal crosslinking is covered with a support layer for supporting the adhesive layer, thus giving a matrix preparation.

The drug used in the pressure-sensitive adhesive layer of the matrix preparation of the present invention is not particularly limited as long as it can percutaneously permeate a biological membrane. Examples of the drug used in the present invention include general anesthetics, hypnotic sedatives, antipyretic/anti-inflammatory analgesics, steroidal anti-inflammatory agents, analeptics/stimulants, anti-motion sickness agents, agents affecting the nervous system, local anesthetics, skeletal muscle relaxants, agents for the autonomic nervous system, antispasmodics, anti Parkinsonism drugs, antihistamines, cardiotonics, antiarrhythmic agents, diuretics, antihypertensives, vasoconstrictors, vasodilators, agents for arteriosclerosis, respiratory stimulants, antitussive/ expectorants, agents for treating peptic ulcers, cholagogues, hormonal drugs, agents for urogenital and anal organs, agents for parasitic skin disease, emollients, vitamin preparations, mineral preparations, hemostatics, anticoagulants, agents for liver disease, agents for habitual intoxication, agents for treating gout, agents for diabetes, antineoplastic agents, radioactive drugs, traditional Chinese preparations, antibiotics, chemotherapeutics, anthelmintics and antiprotozoan agents, and narcotics.

Examples of the antipyretic/anti-inflammatory analgesics include acetoaminophenone, phenacetin, mefenamic acid, diclofenac, fulfenamic acid, aspirin, salicylic acid, aminopyrine, alclofenac, ibuprofen, naproxen, flurbiprofen, ketoprofen, sodium amfenac, epirizole, indomethacin, pentazocine, and piroxicam; and examples of the steroidal anti-inflammatory agents include hydrocortisone, triamcinolone, dexamethasone, betamethasone, and prednisolone.

Examples of the vasodilators include diltiazem, pentaerythritol, isosorbide, trapidil, nicorandil, nitroglycerin, prenilamine, molsidomine, and tolazoline; examples of the antiarrhythmic agents include procainamide, lidocaine, propranolol, alprenolol, atenolol, nadolol, metoprolol, ajmaline, disopyramide, and mexitilen; and examples of the antihypertensives include ecarazine, indapamide, clonidine, bunitrolol, labetalol, captopril, guanabenz, mebutamate, and bethanidine.

Examples of the antitussive expectorants include carbetapentane, chloperastine, oxeladin, clobutinol, clofedanol, noscapine, ephedrine, isoproterenol, clorprenaline, methoxyphenamine, procaterol, tulobuterol, clenbuterol, and ketotifen; examples of the antineoplastic agents include cyclophosphamide, fluorouracil, tegafur, mitomycin C, procarbazine, doxifluridine, and ranimustine; and examples of the local anesthetics include ethyl aminobenzoate, tetracaine, procaine, dibucaine, oxybuprocaine, ambroxol, and propitocaine.

Examples of the hormonal drugs include propylthiouracil, thiamazole, metenolone acetate, estradiol, norethisterone acetate, estriol, and progesterone; examples of the antihistamines include diphenhydramine, chlorpheniramine, promethazine, cyproheptadine, and diphenylpyraline; examples of anticoagulants include potassium warfarin and ticlopidine; examples of the antispasmodics include methylatropine bromide and scopolamine; examples of the general anesthetics include sodium thiopental and sodium pentobarbital; examples of hypnotic sedatives include bromvalerylurea, amobarbital, and phenobarbital; examples of anti-epileptics include phenytoin; and examples of the analeptics and stimulants include methamphetamine.

Examples of the anti-motion sickness agents include difenidol and betahistine; examples of the agents affecting the nervous system include chlorpromazine, thioridazine, meprobamate, imipramine, chlordiazepoxide, and diazepam; examples of the skeletal muscle relaxants include suxamethonium and eperisone; examples of the agents for the autonomic nervous system include neostigmine bromide, and bethanechol chloride; examples of the anti Parkinsonism drugs include pergolide and amantadine; examples of the diuretics include hydroflumethiazide, isosorbide, and furosemide; examples of the vasoconstrictors include phenylephrine; examples of the respiratory stimulants include lobeline, dimorpholamine, and naloxone; and examples of the agents for treating peptic ulcers include glycopyrronium bromide, proglumide, cetraxate, cimetidine, and spizofurone.

Examples of the cholagogues include ursodeoxycholic acid and osalmid; examples of the agents for urogenital and anal organs include hexamine, sparteine, dinoprost, and ritodrine; examples of the agents for parasitic skin disease include salicylic acid, ciclopirox olamine, and croconazole; examples of the emollients include urea; examples of the vitamin preparations include calcitriol, thiamine, sodium riboflavin phosphate, pyridoxine, nicotinamide, panthenol, and ascorbic acid; and examples of the hemostatics include ethamsylate.

Examples of the agents for liver disease include tiopronin; examples of the agents for habitual intoxication include cyanamide; examples of the agents for treating gout include colchicine, probenecid, and sulfinpyrazone; examples of the agents for diabetes include tolbutamide, chlorpropamide, sodium glymidine, glybuzole, buformin, and insulin; examples of the antibiotics include benzylpenicillin, propicillin, cloxacillin, ampicillin, bacampicillin, carbenicillin, cephaloridine, cefoxitin, erythromycin, chloramphenicol, tetracycline, kanamycin sulfate, and cycloserine; examples of the chemotherapeutics include isoniazid, pyrazinamide, and ethionamide; and examples of the narcotics include morphine, codeine phosphate, cocaine, fentanyl, and pethidine.

These drugs can be used singly or in a combination of two or more types, and any form of the drugs such as an inorganic salt or an organic salt can of course be included. The amount of drug added is 0.1 to 30 wt % relative to the total weight of the composition of the pressure-sensitive adhesive layer while taking into consideration a sufficient permeation rate for the patch, irritation of the skin such as reddening, etc.

The pressure-sensitive adhesive layer of the patch of the present invention can contain an absorption promoting agent; the absorption promoting agent that can be used here can be any compound that is conventionally recognized to have a skin absorption promoting effect, and examples thereof include fatty acids, fatty alcohols, fatty acid esters, and ethers having 6 to 20 carbons, aromatic organic acids, aromatic alcohols, aromatic organic acid esters and ethers (those above can be either saturated or unsaturated, and can be cyclic, straight chain, or branched) and, furthermore, lactate esters, acetate esters, monoterpenoid compounds, Azone (trade name), Azone derivatives, glycerol fatty acid esters, sorbitan fatty acid esters (Span (trade name) series) polysorbate types (Tween (trade name) series), polyethylene glycol fatty acid esters, polyoxyethylene hardened castor oil types (HCO series), and sugar fatty acid esters.

Specifically, preferred examples include caprylic acid, capric acid, caproic acid, lauric acid, myristic acid, palmitic acid, stearic acid, oleic acid, linoleic acid, linolenic acid, lauryl alcohol, myristyl alcohol, oleyl alcohol, cetyl alcohol, methyl laurate, isopropyl myristate, myristyl myristate, octyldodecyl myristate, cetyl palmitate, salicylic acid, methyl salicylate, ethylene glycol salicylate, cinnamic acid, methyl cinnamate, cresol, cetyl lactate, ethyl lactate, propyl lactate, geraniol, thymol, eugenol, terpineol, 1-menthol, borneol, d-limonene, isoeugenol, isoborneol, nerol, dl-camphor, glycerol monolaurate, sucrose monolaurate, polysorbate 20, propylene glycol, dipropylene glycol, polyethylene glycol monolaurate, polyethylene glycol monostearate, HCO-60, and 1-[2-(decylthio)ethyl] azacyclopentan-2-one (hereinafter abbreviated as 'pyrrothiodecane'), and particularly preferred examples include lauryl alcohol, 1-menthol, propylene glycol, pyrrothiodecane, dipropylene glycol, and isopropyl myristate.

Such absorption promoting agents can be added at 0.01 to 60 wt %, more preferably 0.1 to 40 wt %, and particularly preferably 0.1 to 20 wt %, relative to the total weight of the composition of the pressure-sensitive adhesive layer, while taking into consideration adequate penetrability as a patch and irritation of the skin such as reddening or edema.

Moreover, if necessary, an antioxidant, a preservative, an ultraviolet-absorbing agent, and an anti-crystallizing agent can be used, and preferred examples of the antioxidant include tocopherol and ester derivatives thereof, ascorbic acid, ascorbic acid stearic acid ester, nordihydroguaiaretic acid, dibutyl hydroxytoluene (BHT), and butyl hydroxyanisole. Preferred examples of the preservative include ethyl paraoxybenzoate, propyl paraoxybenzoate, and butyl paraoxybenzoate. Preferred examples of the ultraviolet-absorbing agent include p-aminobenzoic acid derivatives, anthranilic acid derivatives, salicylic acid derivatives, coumarin derivatives, amino acid compounds, imidazoline derivatives, pyrimidine derivatives, and dioxane derivatives. As the anti-crystallizing agent, polyvinyl pyrrolidone, etc. is preferable. The total amount of such antioxidant, preservative, ultraviolet-absorbing agent, and anti-crystallizing agent can preferably be 15 wt % or less, and more preferably 10 wt % or less, relative to the total weight of the composition of the pressure-sensitive adhesive layer of the patch.

A matrix preparation having the above-mentioned pressure-sensitive adhesive layer can be produced by any method. For example, a drug-containing base composition is melted by heating, applied on a release paper or a support, and then laminated to a support or a release paper to give the present preparation. Alternatively, a drug-containing base component is dissolved in a solvent such as toluene, hexane, or ethyl acetate and spread on a release paper or a support, and after the solvent is removed by drying, the drug-containing base component is laminated to a support or a release paper to give the present preparation. Furthermore, with regard to the matrix preparation of the present invention, as long as the pressure-sensitive adhesive layer comprising the pressure-sensitive adhesive shaped product has the above-mentioned composition containing a boron-containing compound and a drug, any other kinds of configuration and starting materials for the components can be used.

For example, the matrix preparation of the present invention can comprise, in addition to the above-mentioned pressure-sensitive adhesive layer, a support layer for supporting the pressure-sensitive adhesive layer and a release paper layer provided on the pressure-sensitive adhesive layer. A stretchable or non-stretchable support can be employed as the support layer. For example, it can be selected from fabrics, nonwoven fabrics, polyurethane, polyester, polyvinyl acetate, polyvinylidene chloride, polyethylene, polyethylene terephthalate, aluminum sheet, and composite materials thereof.

The present invention is explained below in further detail with reference to Examples of the present invention, but the present invention is not limited to these Examples. In the Examples, '%' means wt % in all cases.

### Preparatory Example 1

| | | |
|---|---|---|
| DURO-TAK® (No. 387-2287) | 4.45 g (solids) | 89% |
| Ethyl acetate solution (solids conc.: 50%) | | |
| Isopropyl myristate | 0.5 g | 10% |
| Boric acid [methanol solution (30 mg/mL)] | 0.05 g | 1% |
| Total | 5.0 g | 100% |

In the above composition, the DURO-TAK® (No. 387-2287, manufactured by National Starch and Chemical Company), which is an acrylic polymer, and isopropyl myristate were mixed, 2 mL of ethyl acetate was added thereto, the mixture was stirred for 1 hour, the methanol solution of boric acid was then added thereto, and the mixture was stirred for 5 minutes to give a pressure-sensitive adhesive layer solution. This was spread out on a silicone-treated surface of an 80 µm thick polyethylene terephthalate (PET) film (release film) and crosslinked at 100°C for 15 minutes to give an 80 µm pressure-sensitive adhesive layer. As a support, a 30 µm thick sand-matted PET film was laminated so that the sand-matted surface was in contact with the pressure-sensitive adhesive layer, thus giving a matrix preparation of the present invention. After the preparation thus obtained was stored at 65°C for 48 hours, the pressure-sensitive adhesive power was measured using a probe tack tester, and it was found that it was 102 gF, which is good. When a 25φ test piece was cut out and affixed to an upper arm and peeled off 2 hours later, there was no residue of the pressure-sensitive adhesive on the skin. These results suggest that the preparation obtained using the pressure-sensitive adhesive of the present invention has the performance of a patch that has appropriate tackiness and cohesive power.

### Example 2

| | | |
|---|---|---|
| DURO-TAK® (No. 387-2287) | 2.9 g (solids) | 58% |
| Estradiol | 0.2 g | 4% |
| Norethisterone acetate | 0.35 g | 7% |
| Isopropyl myristate | 0.5 g | 10% |
| Polyvinylpyrrolidone | 1.0 g | 20% |
| Boric acid [methanol solution (30 mg/mL)] | 0.05 g | 1% |
| Total | 5.0 g | 100% |

In the above composition, the estradiol, norethisterone acetate, isopropyl myristate, and polyvinylpyrrolidone were mixed, 2 mL of ethanol was added thereto, the mixture was stirred for 2 hours, the DURO-TAK® and 1 mL of ethyl acetate were then added thereto and dissolved therein, and the mixture was further stirred for 3 hours until a uniform solution was obtained. The methanol solution of boric acid was added thereto and stirred for 5 minutes to give a pressure-sensitive adhesive layer solution. This was spread out in the same manner as in Example 1, and a support layer was laminated to give a matrix preparation of the present invention. After the preparation thus obtained was stored at 65°C for 48 hours, the pressure-sensitive adhesive power of the preparation was measured using a probe tack tester, and it was found that it was 267 gF, which is good. When a 25 φ test piece was cut out and affixed to an upper arm and peeled off 30 minutes later, there was no residue of the pressure-sensitive adhesive on the skin. The actual measurements of the drug concentrations of this preparation were 100.7% and 100.4% with respect to the initial concentrations of estradiol and norethisterone acetate, suggesting that there was substantially no decomposition of the drugs during the crosslinking reaction. Furthermore, when the stability of this preparation at 40°C was examined, the concentrations after one month were 99.8% and 100.4% relative to the initial concentrations of estradiol and norethisterone acetate respectively, which are good results.

### Comparative Example 1

| | | |
|---|---|---|
| DURO-TAK® (No. 387-2287) | 4.45 g (solids) | 89% |
| Isopropyl myristate | 0.5 g | 10% |
| Boric acid [THF solution (5 mg/mL)] | 0.05 g | 1% |
| Total | 5.0 g | 100% |

In the above composition, when the DURO-TAK® and the isopropyl myristate were mixed and stirred for 1 hour, and the THF solution of boric acid was then added, the viscidity of the mixture greatly increased, and it became impossible to shape it into a sheet shape.

### Comparative Example 2

| | | |
|---|---|---|
| DURO-TAK® (No. 387-2287) | 2.9 g (solids) | 58% |
| Estradiol | 0.2 g | 4% |
| Norethisterone acetate | 0.35 g | 7% |
| Isopropyl myristate | 0.5 g | 10% |
| Polyvinylpyrrolidone | 1.0 g | 20% |
| Boric acid [THF solution (5 mg/mL)] | 0.05 g | 1% |
| Total | 5.0 g | 100% |

In the above composition, the estradiol, norethisterone acetate, isopropyl myristate, and polyvinylpyrrolidone were mixed, 2 mL of THF was added thereto, the mixture was stirred for 2 hours, the DURO-TAK® and 1 mL of ethyl acetate were then added thereto and dissolved therein, and the mixture was further stirred for 3 hours. When the THF solution of boric acid was added thereto, the viscidity of the mixture greatly increased, and it became impossible to shape it into a sheet shape.

### Adhesive power test

The adhesive power was measured as follows. Measurement method: a 1 cm square test piece was cut out of each patch, and a tack value was measured under the conditions below using a probe tack tester (No. 1216S) manufactured by Rigaku Kogyo.
Probe: Bakelite
Adhesion time: 1 sec
Peel speed: 1 mm/sec
Load weight: 200 g

### Drug content test

The drug content was measured as follows. Measurement method: after a 25φ test piece was cut out of each patch, the release paper was removed, the total weight of the pressure-sensitive adhesive layer and the support was measured, this was placed in a 50 mL centrifuge tube, 40 mL of an acetonitrile solution and a 10 mL of a 0.05% acetonitrile solution of benzophenone as an internal standard were added thereto, and the mixture was subjected to ultrasonic extraction for 60 minutes. 0.1 mL of the extract was filtered using a membrane filter and then diluted with 0.9 mL of acetonitrile, and the drug contents of each preparation were calculated from area ratios of estradiol, norethisterone acetate, and the internal standard using high performance liquid chromatography. The preparation from which the drug had been extracted was taken out, the pressure-sensitive adhesive layer was removed from the support and dried, the weight of the support was measured, the weight of the pressure-sensitive adhesive layer was calculated, and the drug concentrations were calculated from these weights and the content of each drug.

### Industrial Applicability

In accordance with the present invention, a pressure-sensitive adhesive layer in a patch, etc. that has satisfactory tackiness and cohesiveness can be shaped into any shape, and a patch that has excellent physical properties such as drug permeability and drug stability can be produced.

## Claims

1. A process for the production of a pressure-sensitive adhesive shaped product comprising a drug, the process comprising mixing, in the presence of a lower alcohol, a hydroxyl group- or carboxyl group-containing polymer and one or more crosslinking agents selected from the group consisting of metal alcoholates and boron-containing compounds, and then shaping either simultaneously with or followed by thermal crosslinking.

2. The process according to Claim 1, wherein the crosslinking agent is dissolved in the lower alcohol in advance.

3. The process according to Claim 1 or 2, wherein the polymer is dissolved in the lower alcohol in advance.

4. The process according to any one of Claims 1 to 3, wherein the crosslinking functional group is a hydroxyl group, and the crosslinking agent is boric acid.

5. The process according to any one of Claims 1 to 4, wherein the drug is added prior to the addition of the crosslinking agent.

6. The process according to any one of Claims 1 to 5, wherein the polymer is an acrylic polymer or a methacrylic polymer.

7. A process for the production of a matrix preparation comprising a drug, the process comprising preparing a pressure-sensitive adhesive shaped product according to one of the Claims 1 to 6, wherein shaping is done by spreading the mixture on a film and the shaping is followed by thermal crosslinking, wherein the crosslinking is either simultaneously with or followed by laminating to a support.

8. A matrix preparation produced by the process according to Claim 7.

9. The matrix preparation according to Claim 8, wherein the pressure-sensitive adhesive shaped product contains substantially no water.

## Patentansprüche

1. Verfahren zur Herstellung eines Haftklebstoff-geformten Produkts, umfassend einen Arzneistoff, wobei das Verfahren das Mischen, in Gegenwart eines niederen Alkohols, eines Hydroxylgruppen- oder Carboxylgruppen-enthaltenden Polymers und eines oder mehrerer Vernetzungsmittel, ausgewählt aus der Gruppe, bestehend aus Metallalkoholaten und Bor-enthaltenden Verbindungen, und das anschließende Formen entweder gleichzeitig mit oder gefolgt von thermischer Vernetzung umfasst.

2. Verfahren nach Anspruch 1, wobei das Vernetzungsmittel vorab in dem niederen Alkohol gelöst wird.

3. Verfahren nach Anspruch 1 oder 2, wobei das Polymer vorab in dem niederen Alkohol gelöst wird.

4. Verfahren nach einem der Ansprüche 1 bis 3, wobei die funktionelle Vernetzungsgruppe eine Hydroxylgruppe ist und das Vernetzungsmittel Borsäure ist.

5. Verfahren nach einem der Ansprüche 1 bis 4, wobei der Arzneistoff vor der Zugabe des Vernetzungsmittels zugesetzt wird.

6. Verfahren nach einem der Ansprüche 1 bis 5, wobei das Polymer ein Acrylpolymer oder ein Methacrylpolymer ist.

7. Verfahren zur Herstellung einer Arzneistoff umfassenden Matrixzubereitung, wobei das Verfahren die Herstellung eines Haftklebstoff-geformten Produkts nach einem der Ansprüche 1 bis 6 umfasst, wobei die Formgebung durch Ausstreichen der Mischung auf einer Folie erfolgt, und im Anschluss an die Formgebung thermische Vernetzung erfolgt, wobei die Vernetzung entweder gleichzeitig mit oder gefolgt von Laminierung auf einen Träger erfolgt.

8. Matrixzubereitung, hergestellt nach dem Verfahren nach Anspruch 7.

9. Matrixzubereitung nach Anspruch 8, wobei das Haftklebstoff-geformte Produkt im Wesentlichen kein Wasser enthält.

## Revendications

1. Procédé de production d'un produit sous forme d'adhésif autocollant comprenant un médicament, le procédé consistant à mélanger, en présence d'un alcool inférieur, un polymère contenant un groupe hydroxyle ou carboxyle et un ou plusieurs agents de réticulation sélectionnés dans le groupe constitué d'alcoolates métalliques et de composés bore, puis à le façonner soit simultanément avec ou suivi d'une réticulation thermique.

2. Procédé selon la revendication 1, en ce que l'agent de réticulation est dissous dans l'alcool inférieur à l'avance.

3. Procédé selon la revendication 1 ou 2, en ce que le polymère est dissous dans l'alcool inférieur à l'avance.

4. Procédé selon l'une des revendications 1 à 3, en ce que le groupe fonctionnel de réticulation est un groupe hydroxyle et l'agent de réticulation est de l'acide borique.

5. Procédé selon l'une des revendications 1 à 4, en ce que le médicament est ajouté avant l'addition de l'agent de réticulation.

6. Procédé selon l'une des revendications 1 à 5, en ce que le polymère est un polymère acrylique ou un polymère méthacrylique.

7. Procédé de production d'une préparation de matrice comprenant un médicament, le procédé consistant à préparer un produit sous forme d'adhésif autocollant selon l'une des revendications 1 à 6, en ce que le façonnage se fait par application du mélange sur un film et le façonnage est suivi d'une réticulation thermique, en ce que la réticulation est soit simultanément avec ou suivie de laminage sur un support.

8. Préparation de matrice produite par le procédé selon la revendication 7.

9. Préparation de matrice selon la revendication 8, en ce que le produit sous forme d'adhésif autocollant ne contient pratiquement pas d'eau.
